Europäisches Patentamt

European Patent Office

Office européen des brevets

(11) Publication number: **0 418 382 A1**

## EUROPEAN PATENT APPLICATION
### published in accordance with Art. 158(3) EPC

(21) Application number: 89903487.0

(22) Date of filing: 20.12.88

(86) International application number: PCT/SU88/00268

(87) International publication number: WO 90/06728 (28.06.90 90/15)

(51) Int. Cl.⁵: A61B 17/36

(43) Date of publication of application: 27.03.91 Bulletin 91/13

(84) Designated Contracting States: CH DE FR GB LI SE

(71) Applicant: NIKOLAENKO, Ivan Grigorievich
pr. Moskovsky, 41-4
Kharkov 310050(SU)

Applicant: GOLOBORODKO, Nikolai Konstantinovitch
pr. Lenina,26-34
Kharkov 310166(SU)

Applicant: KRAVCHENKO, Nina Ivanovna
ul. Selyanskaya,76-207
Kharkov 310011(SU)

Applicant: GOLOBORODKO, Stanislav Konstantinovich
ul. Nikitenko 5-33
Kiev 252139(SU)

Applicant: TJURIN, Alexei Alexandrovich
pr Moskovsky, 124/5a-8
Kharkov 310037(SU)

(72) Inventor: NIKOLAENKO, Ivan Grigorievich
pr. Moskovsky, 41-4
Kharkov 310050(SU)
Inventor: GOLOBORODKO, Nikolai Konstantinovitch
pr. Lenina,26-34
Kharkov 310166(SU)
Inventor: KRAVCHENKO, Nina Ivanovna
ul. Selyanskaya,76-207
Kharkov 310011(SU)
Inventor: GOLOBORODKO, Stanislav Konstantinovich
ul. Nikitenko 5-33
Kiev 252139(SU)
Inventor: TJURIN, Alexei Alexandrovich
pr Moskovsky, 124/5a-8
Kharkov 310037(SU)

(74) Representative: Bibby, William Mark et al
Mathisen, Macara & co. The Coach House
6-8 Swakeleys Road
Ickenham Uxbridge UB10 8BZ(GB)

(54) ELECTROSURGICAL INSTRUMENT.

(57) Electrosurgical instrument comprising a working cutting part (1) on which are mounted insulated current-supplying electrodes (2) carrying cleaning elements. The working cutting part (1) is made of an electroinsulating material and is mounted immovably, whereas the cleaning elements (3) are reciprocally movable in relation to the insulated current-supplying electrodes (2) and are connected with a drive (4).

FIG.1

# ELECTROSURGICAL INSTRUMENT

## Technical Field

The invention relates generally, to medical engineering and more specifically, to electrosurgical instruments for conducting prolonged surgical procedures on various organs and tissues which involve loss of blood,

## Background Art

Electrosurgical instruments making use of a high-frequency current for hemostatic purposes have so far been employed for a rather long period of time. Such instruments are classified, as for a radio-frequency voltage supply method, into monopolar and bipolar, the latter though of a more complicated construction, having much advantage over the monopolar instruments, i.e., higher quality of tissue treatment, no damage to the surrounding tissues, convenience in handling, etc. In this connection monopolar electrosurgical instruments will not hereinafter be the subject of further discussion.

One state-of-the-art electrosurgical instrument is known (cf. GB, J, 2,101,893) to comprise an oblong inner electrode accommodated in a hollow outer electrode and fixed in place by a hollow insulation element interposed between said electrodes. Such an electrosurgical appliance is suitable for coagulation of punctate hemorrhages from a capillary of minor vessel. However, such an instrument is unsuitable for performing extensive surgeries due to too a low power of radio-frequency voltage supplied.

Another prior-art electrosurgical instrument (cf. US, J, 4,228,800) is known to comprise a middle electrode, insulators disposed at the opposite sides of said electrode, and lateral electrodes attached to the insulators symmetrically with respect to said middle electrode.

The aforediscussed appliance is capable of supplying higher-power radio-frequency voltage. However, supply of such high-power currents to the electrodes of said instrument results in carbon deposit on said electrodes appearing as a film of blood and tissue coagulum, which impedes further supply of radio-frequency voltage. If said film is not removed it becomes carbonized or charred in the course of further procedure, thus establishing a dielectric coating on the electrodes, which deteriorates the quality of dissecting action and/or coagulation of the tissues operated upon and results in overheating (thermal burn) of the adjacent tissues and even in failure of the surgery as a whole. Though the tendency towards soiling of the electrode surface by carbon deposition could be reduced due to appropriately selected shape and size of the various components of the instrument, its complete elimination cannot be attained. It is due to the aforesaid fact that the instrument cannot be efficiently used in prolonged surgical procedures, especially in those on parenchymatous organs.

One more electrosurgical instrument now in current use (cf. SU, A, 639,561) is known to comprise a cutting working portion made as two metallic rings situated on a dielectric-material disk, said working portion being associated with a mechanical actuator adapted to impart rotation to the cutting working portion, while electrodes shaped as springy knives are located on the rings.

In the instrument mentioned above the springy knives supply radio-frequency voltage to the ring-shaped electrodes, while the carbon deposit is cut off from the surface of the rings by the same springy knives during rotation of the working portion of the instrument; thus, said knives perform also the part of cleaning elements.

Such an instrument makes it possible to carry out cavity surgeries including those on parenchymatous organs. However, provision of a rotary working component in the instrument makes the cutting and/or coagulation process unstable, since the presence of a movable contact between the disk-shaped electrodes and the springy knives results in opening of the r.f. circuity, sparking and burning-out of the ring-shaped electrodes. Continuous rotation of the cutting working portion with r.f. voltage applied thereto builts up a reactive force on the handle of the instrument, whereby the latter is drawn deeper into the wound, which makes it impossible to carry out accurate dissection and is fraught with a danger of damaging the adjacent tissues. In order to carry out surgery on tissues of differing density and degree of vascularity (such as mucles, the liver, skin, etc.) it is necessary to perform partial dismantling of the instrument concerned with replacement of the disk-shaped cutting working portion thereof. A larger-diamater disk is required for making deep dissections, which in turn involves higher power of the drive and hence increased overall dimensions and weight. On the other hand, short-length dissections require small-diameter disks, whereas to select a disk for short but deep dissections is impossible altogether. It is due to a sophisticated kinematic structure and intricate shape that the instrument cannot be sterilized by conventional mass-scale sterilization techniques; that is why the instrument failed to find widespread application.

## Disclosure of the Invention

It is an object of the invention to provide such an electrosurgical instrument that would make it possible, due to an appropriate arrangement of cleaning elements with respect to current-conducting electrodes and the cutting working portion, to carry out accurate dissections of any tissues within a prolonged operative time, involving minimized loss of blood.

The foregoing and further objects are accomplished due to the fact that in electrosurgical instrument, comprising a cutting working portion, whereon insulated current-carrying electrodes are located, carrying the cleaning elements, according to the invention, the cutting working portion is made from an electrical insulating material and is fixed stationary, while the cleaning elements are capable of moving with respect to the insulated current-carrying electrodes and are connected to the drive.

It is due to the provision of the stationary fixed cutting working portion carrying the stationary fixed electrodes that the herein-proposed electrosurgical instrument is suitable for performing accurate dissections and/or coagulation without any danger of damage to the adjacent tissues. Efficient cleaning of the electrodes by virtue of cleaning elements associated with the drive enables one to conduct surgery within a prolonged period of time at a minimized loss of blood.

The electrosurgical instrument of the invention may comprise a knife located on either of the lateral surfaces of the cutting working portion and interposed between the insulated current-carrying electrodes.

The aforesaid constructional feature makes it possible to efficiently dissect and/or coagulate soft and/or parenchymatous organs due to the use of both lateral surfaces of the cutting working portion.

The knife of the electrosurgical instrument of the invention may be installed on either of the lateral sur faces of the cutting working portion with a possibility of adjusting its projecting portion as for width.

This feature makes it possible to adjust the cutting and/or coagulation rate of soft tissues.

The knife of the electrosurgical instrument of the invention may be linked with the drive which imparts thereto motion with respect to the cutting working portion of the instrument.

This feature makes it possible to dissect and coagulate soft tissues located in hard-of-access places.

It is expedient that a pointed knife-like element be provided on either of the lateral surfaces of the cutting working portion of the instrument.

This feature makes it possible to simplify the construction of the instrument as a whole and to improve sharpening of the knife itself and hence to eliminate carbon deposit accumulation spots round the knife.

It is practicable to provide at least one tooth on the lateral surface of the cleaning element.

This feature makes it possible to attain higher-quality cleaning of the cutting working portion and to reduce its overall dimensions and weight by virtue of a reduced amount of travel performed by the cleaning elements.

It is expedient that the outer surface of the cleaning elements be provided with an electric insulating coating.

This feature makes it possible to concentrate r.f. voltage in the operative zone due to the provision of insulation of the outer surface of the cleaning elements contacting the tissue being operated upon.

It is practicable that the cleaning element of the electrosurgical instrument of the invention be provided with an additional plate arranged in parallel to the surface of the cleaning element and rigidly coupled to the cutting working portion of said instrument.

Provision of such an additional plate makes it possible to conduct surgery on major organs and tissues, since said plate is adapted to take up principal mechanical forces resulting from motion of large amounts of the tissues involved.

It is expedient that the cleaning element be made of a current-conducting material and the additional plate, of an electrical insulating material.

This feature makes it possible to dissect and/or coagulate the tissue being operated upon with that portion of the cleaning element which projects from under the additional plate.

## Summary of the Drawings

In what follows the invention will be illustrated by a detailed description of its specific exemplary embodiments and the accompanying drawings, in which, according to the invention:

FIG. 1 is a general diagrammatic view of an electrosurgical instrument, according to the invention;

FIG. 2 is a section taken along the line II-II in FIG. 1, according to the invention;

FIG. 3 shows an embodiment of the electrosurgical instrument, wherein a knife is provided on one of its lateral surfaces, according to the invention;

FIG. 4 is a section taken along the line IV-IV in FIG. 3, according to the invention;

FIG. 5 is another embodiment of the electrosurgical instrument, wherein the knife is connected to the drive which imparts motion thereto with respect to the cutting working portion, according to the invention;

FIG. 6 is one more embodiment of the electrosurgical instrument, wherein provision is made on one of its lateral surface for a knife made of the material of the cutting working portion, according to the invention;

FIG. 7 is a section taken along the line VII-VII in FIG, 6, according to the invention;

FIG. 8 is still more embodiment of the electrosurgical instrument, wherein at least one tooth is provided on the lateral surface of a cleaning element, according to the invention;

FIG. 9 is yet still more embodiment of the electrosurgical instrument, wherein the cleaning element is provided with an additional plate arranged parallel to the surface of the cleaning element and rigidly, coupled to the cutting working portion, according to the invention;

FIG. 10 is a view facing the arrow A in FIG. 9, according to the invention; and

FIG. 11 is a section taken along the line XI-XI in FIG. 9, according to the invention.

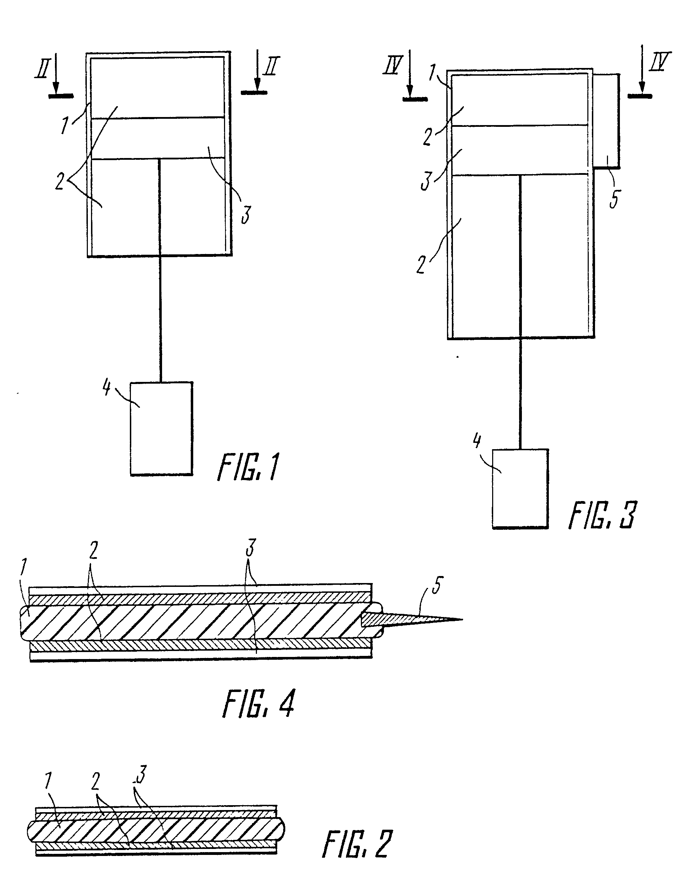

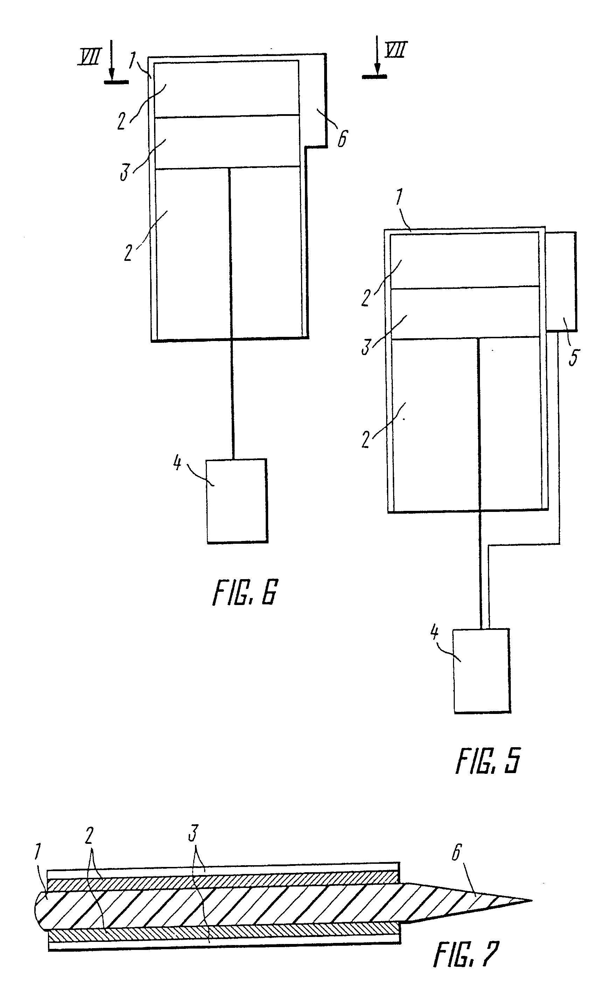

The electrosurgical instrument (FIGS 1, 2) comprises a cutting working portion 1 made of an electrical insulating material and fixed stationary in position. The cutting working portion 1 has lateral surfaces, which are in fact the cutting edges of the instrument and carries electrically insulated current-carrying electrodes 2 mounting cleaning elements 3, each having an outer surface and two lateral surfaces, which are capable of moving with respect to the insulated current-carrying electrodes 2 with the aid of a drive 4.

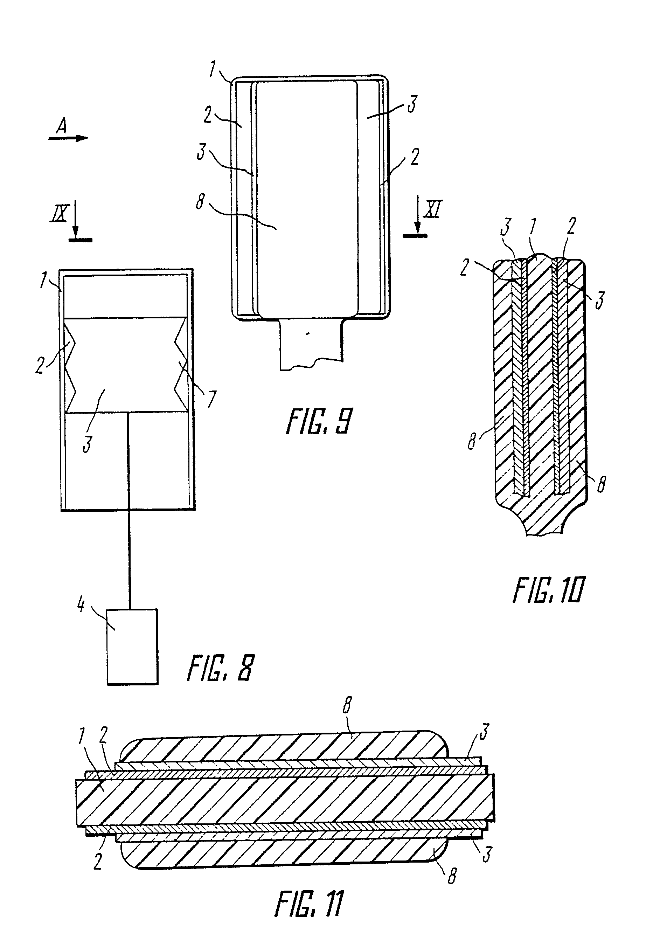

The electrosurgical instrument (FIGS 3, 4) may comprise a knife 5 having a cutting edge and located on one of the lateral surfaces of the cutting working portion 1 between the electrodes 2. Said knife enables one to dissect and/or coagulate both soft tissues (skin, muscles etc.) and parenchymatous organs (the liver, kidneys, etc.)

In cases where surgery is carried out on soft tissues (muscles, etc.), differing in the degree of vascularity with capillaries and minor vessels, it is necessary to change the ratio between the rate of dissection of the tissues involved and the rate of its coagulation. This is attained due to the fact that the knife 5 is mounted on one of the lateral surfaces of the cutting working portion 1 with a possibility of adjusting its projecting portion as for width, In cases of a high degree of vascularity the width of the projecting portion of the knife 5 is adjusted at the minimum, while in cases of a low degree of vascularity, the width of the portion of the knife 5 projecting from the cutting working portion 1 is adjusted at the maximum.

When performing surgery in hard-of-access places, where a possibility of manipulating the instrument is restricted or is absent altogether, it is necessary that the knife should move over the tissue being operated upon. This is ensured due to the fact that the knife 5 (FIG. 5) is connected to the drive 4. The knife 5 can reciprocate lengthwise or crosswise the axis of the instrument, as well as perform swinging motion along an arc, and so on. This enables the instrument to dissect and coagulate tissues without its axial travel.

The same aim is attained in an alternative embodiment of the electrosurgical instrument due to the fact that one of the lateral surfaces of the cutting working portion 1 may have a knife-like pointed end 6 (FIGS 6, 7), which adds to the quality to dissection and coagulation of soft tissues.

In addition, the lateral surface of the cleaning elements 3 may have at least one tooth 7 (FIG. 8) or be serrated, thus adding to the quality of cleaning of the surface of the electrode 2 from carbon deposit and reducing the weight and overall dimensions of the instrument as a whole.

The outer surface of the cleaning elements 3 may have an electrical insulating coating, which enables one to concentrate r.f. voltage at the places of dissection and/or coagulation of the tissues being operated upon.

In the electrosurgical instrument of the invention the cleaning elements 3 may have additional plates 8 (FIGS 9, 10, 11) arranged parallel to the surface of the cleaning elements 3 and rigidly, coupled to the cutting working portion 1, which makes it possible to carry out surgery on major parenchymatous organs.

Provision of the cleaning element 3 made of a current-conducting material and of the additional plates 8 made from an insulating material makes it possible to perform higher-quality dissection and/or coagulation of major organs, especially parenchymatous ones.

The electrosurgical instrument of the present invention is made use of as follows.

During surgery the cutting working portion 1 is brought in contact with the living tissue being operated upon and a voltage is applied from an r.f. generator (omitted in the Drawing) to the electrodes 2, said volrage dissecting and/or coagulating the tissues involved. To prevent a film of carbon deposit from appearing on the surface of the electrodes 2 (FIGS 1, 2) the drive 4 is put in operation to impart motion to the cleaning elements 3 over the surface of the electrodes 2, thus stripping the carbon deposit off the electrode surface. This in turn enables one to get the thus-cleaned surface of the electrodes 2 in a close contact directly with the spot of hemmorrhage (capillaries, minor vessels, etc.) and to coagulate said bleeding spot. Freedom from carbon deposit of the electrodes 2 eliminates thermal effect on the adjacent tissues, since heating of the instrument is considerably reduced. Motion of the cleaning elements 3 over the surface of the electrodes 2 may be reciprocating, swining (along an arc), circular, etc. depending on the kind of the drive being applied. The electrosurgical instrument of the invention is especially efficacious when applied for surgery on parenchymatous organs, where routine surgical techniques result in a vast blood loss (up to 3 or 5 litres) or are impracticable altogheter, e.g., on the liver in the proximity to major blood vessels, and so on.

To carr out surgery on soft tissues (muscles, skin, etc.) where the degree of vascularity is lower than in parenchymatous organs, the instrument may comprise the knife 5 (FIGS 3,4) located on one of the lateral surfaces of the cutting working portion 1 between the current-carrying electrodes 2. This makes it possible to dissect soft tissues, such skin, muscles, etc, in the initial stage of surgery simultaneously with their coagulation with the aid of the cutting working portion 1, viz., with its lateral surface carrying the knife 5. To extend surgery to parenchymatous organs, wherein use of surgical instrument having pointed elements is inadmissible due to a greate number of blood vessels in such organs, use is made of the lateral surface of the cutting working portion 1 devoid of the knife 5. To effect such a transition the instrument is to be merely turned through 180 degrees round its own axis. This makes it possible to use a single instrument during a surgical procedure and carry out dissection and/or coagulation of organs differing in density of tissues, thus minimizing the loss of blood.

In addition, when carrying out surgery on parenchy-matous organs their dissection may be performed without slitting open large- and medium-calibre blood vessels.

The pointed end 6 may be provided on the lateral surface of the cutting working portion 1 (FIGS 6, 7) made of the material of said cutting working portion 1, said pointed end 6 performing the function of a knife blade. This makes it possible to so sharpen the knife edge as to eliminate irregularities (projections or depressions) on the knife edge surface, wherein carbon deposit is liable to accumulate, thus affecting adversely the profile of the cutting edge and hence the quality of dissection made, which in turn is fraught with further hemorrhage. Moreover, such a technical solution makes it possible to simplify the construction of the instrument as a whole.

In performing cavity surgery, especially such that involves vast bleeding areas , it is necessary to have a large cleaned surface of the electrodes 2, for which purpose the cleaning elements 3 should be movable over a considerable length. Such a travel might affect, however, the adjacent tissues and the surgeon's hand, thus deteriorating the cutting process. Provision of a number of the teeth 7 (FIG. 8) on the lateral surface of the cleaning elements 3 makes it possible to reduce the length of travel of the cleaning elements 3 and to improve the quality of cleaning of the surface of the electrodes 2 from carbon deposit. In this case at least one tooth 7 is expedient to be made on the lateral surface of the cleaning elements 3 even in small-sized instruments (as, e.g., for pediatrics) with the cutting working portion 1 and the electrodes 2 of reduced dimensions. Such being the case, optimum adherence of the cleaning element 3 (i.e., its tooth 7) to the electrode 2 is attained.

During surgery it becomes necessary to concentrate r.f. voltage at the surface of the of the electrodes 2 for bringing the latter to the place of coagulation and/or dissection. The cleaning elements 3 are expedient to be made of metal proceeding from technological standpoint. This, however, might result in that r.f. voltage could be applied to the adjacent tissues due to a contact between the surface of the cleaning elements 3 and said tissues, thus inflicting a thermal burn or deteriorating coagulation and/or dissection of the tissues within the operative zone or even ceasing these processes. That is why application of an insulating coating to the outer surface of the cleaning elements 3 makes it possible to concentrate r.f. voltage at the place of coagulation and/or dissection of the tissue involved and to add to the efficacy of practical application of the proposed instrument.

During surgery on majoir organs the liver, stomach, etc., whenever it is necessary to open large-area wound surfaces with the aid of the cutting working portion 1, rather great forces are applied to the outer surface of the cleaning elements 3 so that even with the lateral surface of the cleaning elements 3 made as a number of the teeth 7 the quality of cleaning of the electrodes 2 is impaired, or otherwise the construction of the entire instrument is to be much sophisticated and its overall dimensions and weight be increased considerably. In order to diminish the mechanical forces exerted upon the cleaning elements 3 the latter are provided with the additional plate 8 (FIGS 9, 10, 11) arranged parallel to the surface of the cleaning element 3 and rigidly coupled to the cutting working portion 1.

This makes it possible to relieve mechanically the cleaning elements 3, since the major part of the load involved in displacement of large amounts of the tissue operated upon is taken up by the additional plates 8, while the cleaning elements 3 protruding but for a small length (1 to 2 mm) provide for quality cleaning of the electrodes 2 and take up but inconsiderable forces developed by the tissue being displaced.

When the cleaning elements 3 travel over the surface of the electrodes 2 they cover the effective area of the electrodes 2. To add to the efficacy of coagulation and/or dissection of the tissues involved the cleaning elements 3 are expedient to be made of a current-conducting material. This makes it possible to perform tissue coagulation and/or dissection with the aid of the outer surface of the cleaning element 3 protruding from under the additional plate 8. In this case the cleaning elements 3 get rid of carbon deposit while travelling over the surface of the additional plate 8, whereby the quality of coagulation and/or dissection is enhanced. The cleaning elements 3 may be connected to the respective electrodes 2 through flexible conductors running inside the instrument (omitted in the Drawing). To reduce r.f. voltage scattering b the adjacent tissues the additional plate 8 is expedient to be made of an electrical insulating material.

Industrial Applicability

The electrosurgical instrument of the present invention is adapted for carrying out surgery on the various organs consisting of soft and parenchymatous tissues. The instrument makes it possible to liberate large- and medium-calibre blood vessels from the bulk of the tissue without the risk of damaging their walls. The instrument provides for quality dissection and/or coagulation of tissues with a minimized loss of blood. The operating time is not limited. The instrument is amenable to sterilization by any suitable techniques, including autoclaving, etc.) and is simple in operation and involves no special skill from the part of attending personnel; its application techniques are close to the generally adopted techniques used in operation with a conventional surgical scalpel.

**Claims**

1. An electrosurgical instrument, comprising a cutting working portion (1) with electrically insulated current-carrying electrodes (2) located thereon and carrying cleaning elements (3), characterized in that the cutting working portion (1) is made of electrical insulating material and fixed stationary, and the cleaning elements (3) are installed traversably with respect to the insulated current-carrying electrodes (2) and are associated with the drive (4).

2. An electrosurgical instrument as claimed in Claim 1, characterized in that it comprises a knife (5) which is installed on one of the lateral surfaces of the cutting working portion (1) between the insulated current-carrying electrodes (2).

3. An electrosurgical instrument as claimed in Claim 2, characterized in that the knife is so installed on one of the lateral surfaces of the cutting working portion (1) as to be adjustable for width of its extending cutting edge.

4. An electrosurgical instrument as claimed in Claim 2, characterized in that the knife (5) is associated with the drive (4) imparting motion thereto with respect to the cutting working portion (1).

5. An electrosurgical instrument as claimed in Claim 1, characterized in that one of the lateral surfaces of the cutting working portion (1) has a pointed end (6) shaped as a knife.

6. An electrosurgical instrument as claimed in Claim 1, characterized in that provision is made for at least one tooth (7) located on the lateral surface of the cleaning element (3).

7. An electrosurgical instrument as claimed in Claim 1, characterized in that the outer surface of the cleaning elements (3) has an electrical insulating coating.

8. An electrosurgical instrument as claimed in Claim 1, characterized in that the cleaning elements (3) is provided with an additional plate (8) arranged parallel to the surface of the cleaning element (3) and rigidly coupled to the cutting working portion (1).

9. An electrosurgical instrument as claimed in Claim 8, characterized in that the cleaning element (3) is made of a current-conducting material, while the additional plate (8), of an electrical insulating material.

FIG. 1

FIG. 3

FIG. 4

FIG. 2

FIG. 6

FIG. 5

FIG. 7

A →

IX ↓

1

2

3

3

8

XI ↓

*FIG. 9*

1

2

2

7

3

4

*FIG. 8*

3

1

2

2

8

8

3

*FIG. 10*

8

1

2

3

2

3

8

*FIG. 11*

# INTERNATIONAL SEARCH REPORT

**I. CLASSIFICATION OF SUBJECT MATTER** (if several classification symbols apply, indicate all) *

According to International Patent Classification (IPC) or to both National Classification and IPC

$IPC^5$:     A 61 B 17/36

**II. FIELDS SEARCHED**

| Minimum Documentation Searched [7] | |
|---|---|
| Classification System | Classification Symbols |
| $IPC^4$: | A 61 B 17/32, 17/36, 17/39 |

Documentation Searched other than Minimum Documentation
to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No [13] |
|---|---|---|
| A | JP, B2, 61-28339, 30 June 1986, figure 2 | 1 |
| A | SU, A1, 1366153, (Ju. A. Khitrov et al.) 15 January 1988, the abstract | 2 |
| A | US, A, 4516575 (Cooper Vision, Inc) 14 May 1985, figures 1-2 | 2-4 |
| A | US, A, 3970088 (Valley lab, Inc), 20 July 1976, figures 9,20 | 5 |
| A | US, A, 4534347 (Research Corporation), 13 August 1985, figures 4-6 | 6 |
| | & EP, 150253 | |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 10 July 1989 (10.07.89) | 13 September 1989 (13.09.89) |
| International Searching Authority | Signature of Authorized Officer |
| ISA/SU | |

Form PCT/ISA/210 (second sheet) (January 1985)